# EUROPEAN PATENT APPLICATION

(11) **EP 4 321 478 A1**
(43) Date of publication of application: **14.02.2024**
(21) Application number: 22190307.3
(22) Date of filing: 12.08.2022
(51) Int. Cl.: C01B 3/26, C01B 3/50, C07C 29/151

(54) **PRODUCTION OF SYNGAS FROM CARBON DIOXIDE THROUGH METHANOL**

(71) Applicant: BP P.L.C., London SW1Y 4PD (GB)
(72) Inventor: PATERSON, Alexander James, London, SW1Y 4PD (GB); SUNLEY, Glenn, London, SW1Y 4PD (GB)
(74) Representative: Hamer, Christopher K.

(57) **Abstract**

The present disclosure relates generally to processes for producing syngas. In particular, the disclosure relates to a process comprising: providing a first feed stream comprising H₂ and CO₂; contacting the first feed stream with a hydrogenation catalyst for form a first product stream comprising methanol; providing a second feed stream comprising at least a portion of the methanol of the first product stream; contacting the second feed stream with a methanol decomposition catalyst to form a second product stream comprising syngas.

## Description

### BACKGROUND OF THE DISCLOSURE

### FIELD

The present disclosure relates to integrated processes for the production of syngas from carbon dioxide through a methanol intermediate.

### TECHNICAL BACKGROUND

The conversion of synthesis gas (i.e., a mixture of carbon monoxide and hydrogen, also known as syngas) into hydrocarbons by the Fischer-Tropsch process has been known for decades, but has historically lagged in performance compared to other hydrocarbon synthesis techniques. The growing importance of alternative energy sources has resulted in renewed interest in the Fischer-Tropsch (FT) process as it allows a direct and environmentally-acceptable route to high-quality fuels and feedstock chemicals. Fischer-Tropsch processes use syngas as a starting material, where syngas is a mixture of CO and H₂ gases. Synthesis gas is conventionally produced from fossil fuel sources, primarily through the gasification of coal. However, syngas suffers from poor handling properties, as it must be transported in the low-density vapor phase, or liquefied under high pressure.

FT processes are known for producing linear hydrocarbons for use in fuels, as well as oxygenates that can be useful in fuels and can also serve as valuable feedstock chemicals. The hydrocarbon fuel derived from FT processes is typically better able to meet increasingly stringent environmental regulations compared to conventional refinery-produced fuels, as FT-derived fuels typically have lower contents of sulfur, nitrogen, and aromatic compounds, which contribute to the emission of potent pollutants such as SO₂, NOₓ, and particulates. Products derived from FT processes often have a higher octane rating than hydrocarbons and thus burn more completely, thereby reducing the environmental impact of such a fuel. Alcohols, olefins and other oxygenates obtained may also be used as reagents in other processes, such as in the synthesis of lubricants.

Accordingly, there exists a need to develop improved protocols for the production and handling of syngas.

### SUMMARY

The inventors have identified processes to efficiently convert CO₂ to syngas through the intermediacy of methanol (MeOH). Advantageously, this process can proceed at lower temperature as compared to conventional synthetic routes, and does not require a reverse water-gas shift (rWGS) process.

Thus, in one aspect, the present disclosure provides an integrated process for producing syngas, the method comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion of the methanol to form a second product stream comprising syngas (e.g., comprising CO and H₂).

Other aspects of the disclosure will be apparent to those skilled in the art in view of the description that follows.

### BRIEF DESCRIPTION OF THE DRAWINGS

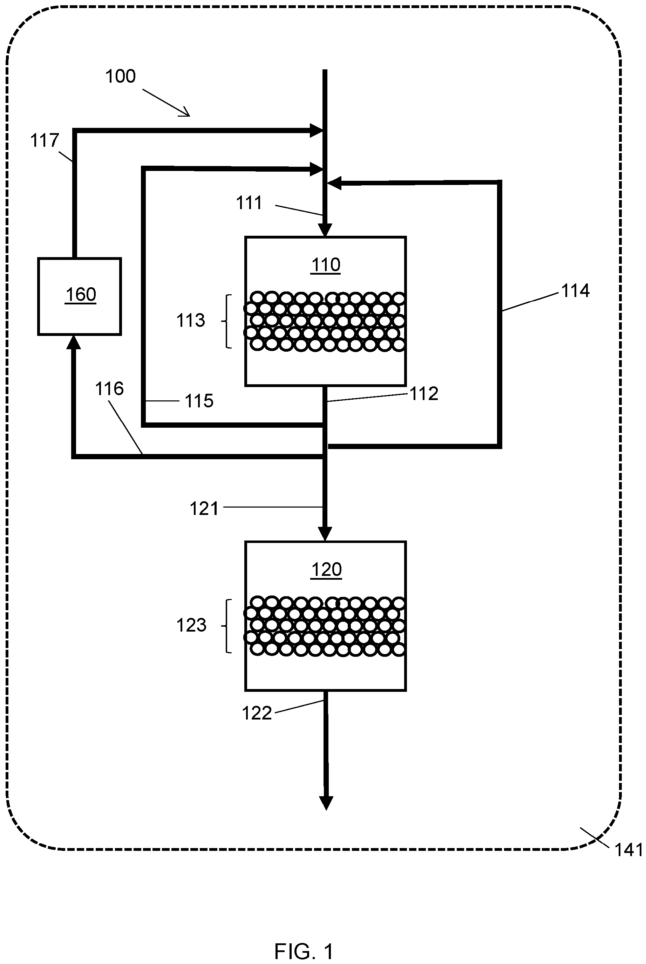

FIGS. 1-4 provide process schematics according to example embodiments of the disclosure.

### DETAILED DESCRIPTION

The present disclosure relates to techniques for preparing syngas from carbon dioxide. Carbon dioxide is a widely available gas (currently present in the atmosphere at about 400 ppm) that is inert to many transformations. Additionally, the tendency of carbon dioxide to absorb infrared radiation has led to its designation as a greenhouse gas. Thus, there is a need to develop economical processes that utilize carbon dioxide, especially waste carbon dioxide that would otherwise be added to the ever-rising concentration of carbon dioxide in the atmosphere. Carbon dioxide is produced by many industries where it can be captured as a point source to be used as a feedstock, for example from facilities involved in steel production, fossil fuel power generation, cement production, fermentation processes or fertilizer production. Carbon dioxide may also be sourced from direct air capture projects. Advantageously, processes that use syngas, such as Fischer-Tropsch hydrocarbon synthesis, that use waste carbon dioxide as a feed have the potential to be low-carbon, carbon neutral, or even have a negative carbon footprint. One way to achieve this is to transform carbon dioxide and hydrogen into methanol. Methanol, being a liquid at ambient temperatures and pressures, may optionally be transported through low-pressure pipelines, ships or trucks, and thus generally allows for increased transportation efficiency as a liquid. Methanol may then be subjected to a methanol decomposition step in order to generate H₂ and CO in a 2:1 ratio. Mixtures of H₂ and CO are commonly referred to as "syngas" and find wide-ranging utility. At least the CO of the methanol decomposition product can then be utilized in a Fischer-Tropsch synthesis reaction in order to generate hydrocarbons; the H₂ from the methanol decomposition is desirably also used in the Fischer-Tropsch synthesis. For example, some Fischer-Tropsch processes operate at a H₂:CO ratio of 2:1 or greater, such at 2.2:1 or 3:1. In such cases, the H₂ and CO mixture from methanol decomposition may be supplemented by a hydrogen source, such as from a recycle stream or green hydrogen produced with renewable energy, to form the Fischer-Tropsch reaction mixture.

Accordingly, in one aspect, the present disclosure provides an integrated process for producing syngas, the method comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion the methanol to form a second product stream comprising syngas (e.g., comprising CO and H₂).

As used herein, a "feed stream" is used to mean the total material input to a process step, e.g., CO₂ hydrogenation or methanol decomposition, regardless of whether provided in a single physical stream or multiple physical streams, and whether through a single inlet or multiple inlets. For example, H₂ and CO₂ of the first feed stream can be provided to a hydrogenation reactor in a single physical stream (e.g., in a single pipe to the reactor), or in multiple physical streams (e.g., separate inlets for CO₂ and H₂, or one inlet for fresh CO₂ and H₂ and another for recycled CO₂ and/or H₂). Similarly, a "product stream" is used to mean the total material output from a process step, e.g., hydrogenation of carbon dioxide or methanol decomposition, regardless of whether provided in a single physical stream or multiple physical streams, and whether through a single reactor outlet or multiple reactor outlets.

As used herein, the term "syngas" refers to a mixture of CO and H2, wherein CO and H2 are present in a molar ratio in the range of 0.2:1 to 10:1, and the combined CO and H2 comprise between 10 vol% and 80 vol% of the mixture. Syngas is gaseous at standard temperature and pressure, but may be liquefied at elevated pressures and/or decreased temperatures.

The first feed stream comprises H₂ and CO₂ for subsequent contacting with a hydrogenation catalyst for the production of methanol. Accordingly, in various embodiments as otherwise described herein, the first feed stream includes at least 10 mol% H₂. For example, in various embodiments, the first feed stream includes at least 20 mol% H₂, e.g., at least 30 mol% H₂. In various embodiments as otherwise described herein, the first feed stream includes at least 5 mol% CO₂. For example, in various embodiments, the first feed stream includes at least 10 mol% CO₂, e.g., at least 15 mol% CO₂.

In general, CO₂ hydrogenation to produce methanol proceeds according to the reaction: CO₂ + 3H₂ → CH₃OH + H₂O. Accordingly, in various embodiments as otherwise described herein, the ratio of H₂:CO₂ of the first feed stream is at least 1:1, e.g., at least 1.5:1, on a molar basis. For example, in various embodiments as otherwise described herein, the ratio of H₂:CO₂ of the first feed stream is at least 2:1, e.g., at least 2.5:1, or at least 3:1, or at least 4:1, or at least 5:1, or at least 6:1, or at least 8:1. In particular embodiments, the ratio of H₂:CO₂ of the first feed stream is no more than 25:1, e.g., no more than 20:1, or no more than 15:1, or 12:1.

In some embodiments, the first feed stream comprises other gases in addition to CO₂ and H₂. For example, in various embodiments, the first feed stream further comprises one or more of CO, CH₄, and N₂. For example, in particular embodiments, the first feed stream comprises other gases in addition to CO₂ and H₂ (e.g., one or more of CO, CH₄, and N₂) in an amount up to 70 mol%. In various embodiments, the first feed stream comprises no more than 1% O₂, for example, no more than 0.1% O₂, or no more than 0.01% O₂, or substantially no O₂.

As otherwise described herein, the first feed stream is contacted with a hydrogenation catalyst. The hydrogenation catalyst may be selected by the person of ordinary skill in the art. Examples of suitable catalysts include Cu/ZnO catalysts, for example supported on aluminum oxide or zirconium oxide.

Advantageously, the CO₂ hydrogenation reaction may be performed at a relatively low temperature, leading to increased energy efficiency and integration of the overall process. For example, in various embodiments as otherwise described herein, the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. In various embodiments, the contacting of the first feed stream with the hydrogenation catalyst is performed at a pressure of no more than 100 bar, for example, no more than 80 bar, or no more than 60 bar.

As described herein, the hydrogenation of CO₂ advantageously produces methanol with high selectivity. Accordingly, in various embodiments as otherwise described herein, the hydrogenation of CO₂ is performed with a selectivity of at least 50% for methanol, e.g., at least 55%, or at least 60%. For example, in particular embodiments, the hydrogenation of CO₂ is performed with a selectivity of at least 65% for methanol (e.g., at least 70%, or at least 75%, or at least 80% for methanol). Advantageously, the hydrogenation of CO₂ can be performed with a low selectivity for methane. In various embodiments as otherwise described herein, the hydrogenation of CO₂ is performed with a selectivity of no more than 20% for methane, e.g., no more than 10%, or no more than 5% for methane. As the person of ordinary skill in the art will understand, "selectivity" for a given species is the fraction of the carbon-containing reactant that reacts that is converted to that species, i.e., not counting unreacted material.

Importantly, the CO₂ hydrogenation reaction as described herein is not a reverse water-gas shift reaction. As known in the art, the reverse water-gas shift reaction transforms CO₂ and H₂ into CO and H₂O. Accordingly, in various embodiments as otherwise described herein, the hydrogenation of CO₂ is performed with a selectivity of no more than 20% for CO, e.g., no more than 10%, or no more than 5% for CO. In particular embodiments, the hydrogenation of CO₂ is performed with a selectivity of no more than 2% for CO, or 1% for CO.

During the CO₂ hydrogenation reaction, at least a portion of the CO₂ is hydrogenated into MeOH and H₂O. Advantageously, this process may be performed with a relatively high conversion of CO₂. Accordingly, in various embodiments as otherwise described herein, the hydrogenation of CO₂ is performed with a conversion of CO₂ of at least 25%, e.g. at least 35%, e.g., at least 45% or at least 50%.

The person of ordinary skill in the art will be familiar with catalytic methods for the hydrogenation of carbon dioxide to methanol. Various examples of catalysts and catalytic processes are described in R.Guil-Lopez et al., Materials, 12, 3902 (2019); Xiao et al., In: Aresta et al. (eds) An Economy Based on Carbon Dioxide and Water (2019); Marlin et al., Front. Chem. (2018); Rodriguez et al., ACS Cat. 5(11), 6696 (2015); Choundhury, Chem. Cat. Chem. 4(5), 609 (2012), each of which is hereby incorporated herein by reference in its entirety.

The CO₂ hydrogenation produces a first product stream. In various embodiments as otherwise described herein, the first product stream includes at least 15 mol% methanol, e.g., at least 25 mol%, or at least 35 mol% methanol. As described herein, the CO₂ hydrogenation also has advantageously low selectivity for other products such as methane and/or carbon monoxide. Accordingly, in various embodiments as otherwise described herein, the first product stream includes no more than 10 mol% methane, e.g., no more than 5 mol% methane, or no more than 2 mol% methane. In various embodiments as otherwise described herein, the first product stream includes no more than 10 mol% CO, e.g., no more than 5 mol% CO, or no more than 2 mol% CO. Of course, in some embodiments, there may be more methane and/or CO present, for example, when provided as part of the first feed stream. As would be understood by the skilled person, the composition of the first product stream as disclosed herein is calculated exclusive of inert gas or unreacted CO₂ and/or H₂.

As described herein, the CO₂ hydrogenation step produces both methanol and water. In some embodiments, the formed water can be deleterious to subsequent processes. For example, excess water present in the methanol decomposition process step can undesirably cause CO to be converted back to CO₂ through the water-gas shift reaction. Additionally, adventitious water may react with methanol in a methanol steam reforming reaction to form CO₂ and hydrogen, undermining process efficiency. Accordingly, in various embodiments as otherwise described herein, the process further comprises separating at least a portion of water from the first product stream. For example, in particular embodiments, the process further comprises separating at least 50%, or at least 75%, or at least 90%, or at least 95%, or at least 99%, or at least 99.5% of the water in the first product stream. In various embodiments as otherwise described herein, the portion of the first product stream that is included in the second feed stream has a water content of no more than 10 mol%, e.g., no more than 2 mol%, or no more than 1 mol%, or no more than 0.5 mol%.

In embodiments in which water is separated from the CO₂ hydrogenation step, the water may be disposed of as waste, or recycled into other processes. For example, in various embodiments, the separated water is directed to an electrolysis reactor for the formation of H₂ gas, e.g., for use in this integrated process or in other processes. In certain embodiments, the separated water is subjected to a purification step before introduction into the electrolysis reactor.

In various embodiments as otherwise described herein, not all H₂ gas input to the CO₂ hydrogenation is reacted. Accordingly, in such embodiments, the process may further comprise separating at least a portion of H₂ from the first product stream. For example, in particular embodiments as otherwise described herein, the process further comprises separating at least 50%, or at least 60%, or at least 75%, or at least 90%, or at least 95% of the H₂ in the first product stream. The separated H₂ may be optionally purified, and then optionally recycled. In various embodiments, the at least a portion of the separated H₂ may be recycled to the first feed stream, and/or may be directed to another reactor. For example, in various embodiments, the at least a portion of the separated H₂ is directed to a Fischer-Tropsch reactor feed stream. In particular embodiments, the process further comprises activating a Fischer Tropsch catalyst using at least a portion of the H₂ separated from the first product stream. Additionally or alternatively, at least a portion of the H₂ separated from the first product stream may be used to activate a methanol decomposition catalyst.

Advantageously, some processes described herein may be integrated for increased efficiency. For example, in various embodiments as otherwise described herein, the contacting of the second feed stream with the methanol decomposition catalyst and the contacting of the first feed stream with the hydrogenation catalyst are performed in the same plant. As would be appreciated by the person of ordinary skill in the art, the "same plant" signifies that the two processes occur within a small geographical area or property, so that it would be economical to directly connect the two without a significant intervening transportation step. For example, in particular embodiments as otherwise described herein, at least a portion of methanol of the first product stream may be provided directly to the second feed stream.

As described herein, there are a number of uses of hydrogen from the first product stream. Any or all of them may be exploited in a particular real-world process. Notably, the inventors recognize that the presence of hydrogen in the second feed stream may work against the conversion of methanol to carbon monoxide (i.e., because hydrogen is a co-product in that reaction, presence of hydrogen in the second feed stream would force equilibrium in the undesired reverse direction). Accordingly, in various embodiments, the portion of the first product stream that is provided to the second feed stream includes no more than 50% of the hydrogen of the first product stream, e.g., no more than 25%, no more than 10%, or no more than 5% of the hydrogen of the first product stream.

The portion of the first product stream that is provided to the second feed stream may be advantageously be at elevated temperature and/or pressure. This may reduce capital costs by mitigating the heating and/or pressurization needs of the second feed stream. Accordingly, in various embodiments as otherwise described herein, the portion of the first product stream that is provided to the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 C, or 300-400 °C. In particular embodiments, the portion of the first product stream that is provided to the second feed stream is at a pressure of 0 to 50 barg, e.g., 1 to 50 barg.

Compared to gaseous compositions, compositions that are liquid at ambient temperature and pressure generally possess lower capital expenditure for handling due to their increased density and decreased need for high pressures and/or low temperatures. The present inventors have noted that the methanol product of the CO₂ hydrogenation can be simply stored and/or transported. Accordingly, in various embodiments as otherwise described herein, the contacting of the second feed stream with the methanol decomposition catalyst and the contacting of the first feed stream with the dehydrogenation catalyst are performed in different plants. As used herein, a different "plant" is not merely a different catalyst bed or different vessel, but rather a different facility than the facility in which the carbon dioxide hydrogenation is performed. In such embodiments, the plant can be, e.g., at least 1 km away from the facility in which the hydrogenation is performed. In various such embodiments, the methanol from the first product stream may be stored and/or transported before provision to the second feed stream.

In various embodiments as otherwise described herein, at least a portion of the methanol of the first product stream is stored (e.g., in one or more tanks) before provision to the second feed stream. The storage may be for any suitable time. In various embodiments, the stored portion of the methanol of the first product stream is stored at least one day, e.g., at least two days, or at least three days, or at least 1 week. In particular embodiments, the stored portion of the methanol is transported at least 1 km during storage, e.g., at least 2 km, or at least 10 km. In some embodiments, the methanol may be transported via pipeline.

The second feed stream introduces methanol to for decomposition into CO and H₂. Methanol decomposition is a distinct process from methanol reforming. In methanol reforming, methanol and water are reacted to produce CO₂ and H₂. Accordingly, in various embodiments as otherwise described herein, methanol reforming is not utilized. For example, in particular embodiments, no more than 10% of the H₂ is derived from methanol reforming (e.g., no more than 5%, or no more than 1%).

The water-gas shift reaction is another method to generate H₂ by reacting carbon monoxide with water. The reaction is reversible as well, where carbon dioxide may be reacted with hydrogen to form carbon monoxide and water. Both of these reactions are conventionally used in the art. Advantageously, the presently disclosed process avoids the use of both the water-gas shift reaction and reverse water-gas shift reaction. Accordingly, in various embodiments as otherwise described herein, less than 10% (e.g., less than 5%, or less than 2%) of each of the H₂, CO, and CO₂ of the process are generated by the water-gas shift reaction or reverse water-gas shift reaction, as appropriate. In various processes as disclosed herein, there is no use of a dedicated water-gas shift reactor or reverse water-gas shift reactor (however, as would be appreciated by the person of ordinary skill in the art, the water-gas shift reaction or reverse water-gas shift reaction can operate to a minor degree as a side reaction during certain processes).

As described herein, methanol decomposition is utilized to form a product stream that includes CO and H₂. Accordingly, in various embodiments as otherwise described herein, the second feed stream comprises at least 5 mol% methanol. For example, in particular embodiments, the second feed stream comprises at least 7.5% methanol, e.g., at least 10% methanol, or at least 15% methanol, or at least 20% methanol, or at least 25 mol% methanol.

Optionally, the second feed stream may also comprise one or more additional gases, which may be inert or reactive. In such embodiments, the second feed stream may further comprise one or more of H₂, CO, CH₄, CO₂, and N₂. In particular embodiments, the second feed stream comprises an inert carrier gas, wherein the inert carrier gas includes one or more of CH₄, CO₂ and N₂. Additionally or alternatively, H₂ and/or CO may be added to the second feed stream in order to tune the decomposition reaction. In such embodiments, the second feed stream further comprises H₂ and/or CO. In various embodiments as otherwise described herein, one or more of H₂, CO, CH₄, CO₂, and N₂ are be present in second feed stream an amount in the range of up to 50 mol%, e.g., up to 40 mol%, or up to 30 mol%.

In various embodiments, the second feed stream has a low water content. Any water present can react with CO to be at least partially converted to CO₂ and H₂ through the water-gas shift reaction, and water can also react with methanol via reforming to provide H₂ and CO₂. The generated H₂, according to the reaction equilibrium of methanol decomposition, would then disfavor the decomposition of methanol to CO and H₂. Accordingly, in various embodiments as otherwise described herein, the second feed stream has a concentration of water of no more than 5 mol%, e.g., no more than 2 mol%, or no more than 1 mol %, or no more than 0.5 mol%, or no more than 0.3 mol%, or no more than 0.2 mol%, or no more than 0.1 mol%.

The second feed stream may be provided at elevated temperature. For example, in various embodiments as otherwise described herein, the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.

The second feed stream is contacted with a methanol decomposition catalyst. Any suitable decomposition catalyst may be used; a variety are known in the art. For example, the decomposition catalyst may include a transition metal, for example, Ni, Co, Rh, Ir, Cu, Pt, Ru, or mixtures thereof. The decomposition catalyst may be a supported catalyst, wherein the support is a refractory oxide, such as oxidized diamond, silica, zirconia, ceria, titania, alumina, or magnesia. In some embodiments, the methanol decomposition catalyst is a copper/zinc oxide catalyst, e.g., on alumina.

The contacting of the second feed stream with the methanol decomposition catalyst occurs at a temperature suitable to effect efficient methanol decomposition. In various embodiments as otherwise described herein, the contacting of the second feed stream with the methanol decomposition catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C. The contacting may be performed at a variety of suitable pressures, for example, in the range of 0 to 50 barg.. Accordingly, in such embodiments, the contacting with the methanol decomposition catalyst may be performed at relatively high pressures, for example, in the range of up to 50 barg, e.g., 20 to 40 barg.

As described herein, the contacting of the second feed stream with the methanol decomposition catalyst may be performed in the same plant as the contacting of the first feed stream with the hydrogenation catalyst. In such examples, it may be advantageous to coordinate the temperatures of the methanol decomposition reaction and the hydrogenation reaction in order to avoid excessive heating and cooling capital costs and energy costs. Accordingly, in particular embodiments as otherwise described herein, the contacting of the second feed stream with the methanol decomposition catalyst is performed at a temperature within 75 °C of a temperature of the contacting of the first feed stream with the hydrogenation catalyst, e.g., within 50 °C.

The methanol decomposition reaction generates CO and H₂, ideally with minimal other products. Accordingly, in various embodiments as otherwise described herein, the decomposition of methanol is performed with a carbon product selectivity of at least 50% for CO, e.g., at least 60%, or at least 70%, or at least 80%, or at least 90% for CO. Advantageously, in various embodiments, the decomposition of methanol is performed with a selectivity of no more than 20% for CO₂, e.g., no more than 15%, or no more than 10%, or no more than 5%. In various embodiments as otherwise described herein, the decomposition of methanol is performed with a conversion of methanol of at least 30%, e.g., at least 40%, or at least 50%, or at least 60%, or at least 65%, or at least 70%, or at least 75%.

As described herein, the decomposition of methanol generates CO and H₂. Accordingly, in various embodiments as otherwise described herein, the second product stream comprises at least 20 mol% total of CO and H₂, e.g., at least 35 mol%, or at least 50 mol%, or at least 65 mol%. Without wishing to be bound by theory, methanol decomposition under these conditions is expected to generated two moles of H₂ per mole of CO. Accordingly, in various embodiments as otherwise described herein, the second product stream has a molar ratio of hydrogen to carbon monoxide in the range of 0.5:1 to 5:1, e.g., 1:1 to 3:1, or 1.5:1 to 2.5:1, or 1.5:1 to 3.5:1. Of course, in other embodiments, this molar ratio may be different, e.g., as a consequence of inclusion of H₂ or CO in the second feed stream.

Advantageously, a proportion of methanol is consumed during the methanol decomposition reaction. Accordingly, in various embodiments as otherwise described herein, the second product stream includes no more than 75 mol% methanol, e.g., no more than 60 mol% methanol, no more than 50 mol% methanol, or no more than 25 mol% methanol.

In some embodiments, further removal of methanol from the second product stream is desired. For example, in various embodiments as otherwise described herein, the process further comprises separating at least a portion of methanol from the second product stream, e.g., separating at least 50%, or at least 75%, or at least 90%, or at least 95% of the methanol from the second product stream. Advantageously, methanol of the second product stream, e.g., at least a portion of the methanol separated from the second product stream, may be transferred or recycled to other processes. For example, in various embodiments as otherwise described herein, the process further comprises recycling to the second feed stream at least a portion of the methanol separated from the second product stream.

The person of ordinary skill in the art will be familiar with catalytic methods for the decomposition of methanol to CO and H₂. Various examples of catalysts and catalytic processes are described in U.S. Patent no. 6,541,142, U.S. Patent no, 9,883,773, and U.S. Patent no. 4,716,859 each of which is hereby incorporated herein by reference in its entirety.

As described herein, the methanol decomposition reaction produces CO and H₂ in a theoretical 2:1 molar ratio, although the actual reaction output may vary. This can result in excess hydrogen for subsequent processes, depending on the desired ratio. And, notably, hydrogen may be present in the second feed stream, further increasing the ratio in the second product stream. Accordingly, in various embodiments as otherwise described herein, the process further comprises separating at least a portion of H₂ from the second product stream. The separated H₂ may be suitable for a variety of processes. For example, in particular embodiments, at least a portion of the separated H₂ from the second product stream is provided to the first feed stream. Additionally or alternatively, in various embodiments as otherwise described herein, the process further comprises activating the methanol decomposition catalyst using at least a portion of the H₂ separated from the second product stream.

The present inventors have noted that carbon dioxide is a chief input of carbon to the processes described herein, and that carbon dioxide can advantageously be provided by a variety of sources. Importantly, at least part of the carbon dioxide of the first feed stream (e.g., at least 50%, at least 75%, at least 90% or at least 95%) can come from a renewable source.

Carbon dioxide is a common waste material, and often desirable to be removed from waste streams rather than be vented to the atmosphere. Such capture of carbon dioxide is critical to the implementation of many renewable initiatives as it serves to lower the carbon footprint of the associated process. Advantageously, the carbon dioxide utilized in the processes described herein may be carbon dioxide collected from the atmosphere or that would otherwise have been released into the atmosphere, e.g., from a combustion or other industrial process. The carbon dioxide may be captured, where it is collected or absorbed after release from an industrial process, or harvested directly from the atmosphere. Methods of carbon dioxide capture are known to those of skill in the art. In various embodiments, at least a portion of the CO₂ of the first feed stream is from direct air capture. Additionally or alternatively, carbon dioxide is often scrubbed from industrial effluent, especially processes that generate large amounts of carbon dioxide as a byproduct. Accordingly, in various embodiments as otherwise described herein, at least part of the CO₂ of the first feed stream (e.g., at least 50%, at least 75%, at least 90% or at least 95%) is captured from a manufacturing plant, e.g., a bioethanol plant, a steel plant or a cement plant.

As noted above, renewable sources can be used to provide CO₂ to the claimed processes. For example, biomass is an attractive source of renewable carbon dioxide for use in the processes described herein. One source of biomass is agricultural products in the form of dedicated energy crops such as switchgrass, miscanthus, bamboo, sorghum, tall fescue, kochia, wheatgrass, poplar, willow, silver maple, eastern cottonwood, green ash, black walnut, sweetgum, and sycamore. Another biomass source is agricultural waste or agricultural crop residue. Conventional agricultural activities, including the production of food, feed, fiber, and forest products, generate large amounts of waste plant material. Examples of such materials include corn stover, wheat straw, oat straw, barley straw, sorghum stubble, and rice straw. A third biomass source is through forestry residues left after timber operations. Biomass may also be in the form of commercial waste, industrial waste, sewage sludge, and municipal waste, which includes commercial and residential garbage, including yard trimmings, paper and paperboard, plastics, rubber, leather, textiles, and food waste. Accordingly, in various embodiments as otherwise described herein, the at least a portion of the CO₂ of the first feed stream is derived from a renewable source. For example, in particular embodiments, at least a portion (e.g., at least 50%, at least 75%, at least 90% or at least 95%) of the CO₂ of the first feed stream is derived from biomass, for example, agricultural biomass or municipal waste biomass. Additional sources of agricultural biomass will be apparent to one of skill in the art as dictated by local availability, economics, and process compatibility.

To generate carbon dioxide from a carbon-containing material, such as biomass, the material is typically subjected to gasification. Gasification involves heating the material under controlled conditions to generate gaseous streams of carbon monoxide, hydrogen, and carbon dioxide. Controlled amounts of other reactants, such as oxygen and/or steam, may be used to tune the process. Gasification conditions are tuned in accordance with the carbon-containing material being gasified in order to efficiently produce gaseous products. In various embodiments, the carbon dioxide of the first feed stream includes carbon dioxide from gasification of biomass, e.g., at least 50%, at least 75%, at least 90% or at least 95% of the carbon dioxide is from gasification of biomass. The biomass may be any source as described above, or from multiple sources may be combined.

Conventional H₂ gas is most commonly derived from natural gas, often through steam reforming of methane, hydrocarbon partial oxidation, and/or coal gasification. However, each of these methods, as conventionally performed, release significant amounts of CO₂ into the atmosphere and rely on fossil fuels as a starting material and/or energy source. In contrast, other sources of H₂ are known which are preferable from an environmental standpoint. Accordingly, in various embodiments as otherwise described herein, at least a portion of the H₂ of the first feed stream and/or the second feed stream (e.g., at least 50%, at least 75%, at least 90% or at least 95%) is from a renewable resource.

As known in the art, hydrogen can be provided with color-based names according to its source of production. Examples of types of hydrogen include green hydrogen, blue hydrogen, grey hydrogen, black hydrogen, brown hydrogen, pink hydrogen, turquoise hydrogen, yellow hydrogen, and/or white hydrogen.

One potential source of green hydrogen is through the electrolysis of water. Numerous methods of electrolysis are known in the art. For example, electrolysis may be performed on pure water to produce hydrogen gas and oxygen gas, or on other solutions, such as saline solution, to produce hydrogen gas and another product (e.g., chlorine gas). In particular embodiments, the hydrogen is formed through the electrolysis of a saline solution. Water electrolysis is described further in U.S. Patent No. 4,312,720, U.S. Patent No. 4,021,323, and U.S. Patent No. 4,094,751, each of which is incorporated by reference in their entirety.

To qualify as green hydrogen, the electrical power used for the water electrolysis must be from a renewable source, that is, a source that does not depend on fossil fuel combustion. Example sources of renewable power include solar power through photovoltaic capture or solar thermal technology, wind power, geothermal energy capture, hydroelectric energy, or other renewable sources. Hydrogen that uses solar power for water electrolysis is sometimes called yellow hydrogen. Appropriate renewable energy sources are known to those of skill in the art, and may optionally be selected through certification by an appropriate agency. Accordingly, in various embodiments as otherwise described herein, at least a portion of the H₂ of the first feed stream is green hydrogen and/or yellow hydrogen. For example, in particular embodiments, the process further comprises providing at least a portion of the H₂ of the first feed stream by electrolysis of water. In various embodiments, the electrolysis of water is performed using electricity at least partially derived from a renewable source.

Blue hydrogen is defined as hydrogen gas produced with some reliance on fossil fuels, but in a process that is overall carbon neutral (i.e., does not result in any net introduction of carbon dioxide into the atmosphere). In various embodiments as otherwise described herein, the hydrogen utilized in the processes as otherwise described herein comprises blue hydrogen. An example of blue hydrogen is hydrogen gas produced from fossil fuel-derived hydrocarbons such as methane gas, wherein the resulting carbon product is captured or otherwise utilized. For example, steam reforming of methane may be conducted to produce three moles of hydrogen gas and one mole of carbon monoxide for each mole of methane. Methane steam reforming is highly endothermic, requiring significant energy input. Of course, the energy required to perform these processes must be sources from renewable sources, or sources with adequate carbon capture technology. Accordingly, in various embodiments as otherwise described herein, at least a portion of the hydrogen (e.g., at least 50%, at least 75%, at least 90% or at least 95%) is formed through the steam reforming of methane. Steam reforming of methane to produce hydrogen is discussed in International Patent Application Publication no. 2004/022480, which is herein incorporated by reference in its entirety. Accordingly, in various embodiments as otherwise described herein, at least a portion of the H₂ of the first feed stream is blue hydrogen.

Grey hydrogen is the source of most conventional H₂. Grey hydrogen is created from natural gas/methane through steam reforming, but, in contrast to blue hydrogen, none of the resulting greenhouse gases (e.g., CO₂) is captured. In various embodiments as otherwise described herein, at least a portion of the H₂ of the first feed stream is grey hydrogen.

Black hydrogen and brown hydrogen are produced from black coal or brown coal, respectively, often through gasification thereof without any carbon capture. In various embodiments as otherwise described herein, at least a portion of the H₂ of the first feed stream is black hydrogen and/or brown hydrogen.

Pink hydrogen is generated through water electrolysis where the required energy for the electrolysis is generated by nuclear power. In various embodiments as otherwise described herein, at least a portion of the H₂ of the first feed stream is pink hydrogen. Pink hydrogen is also known as purple hydrogen or red hydrogen.

Turquoise hydrogen is generated by methane pyrolysis, where the byproducts are hydrogen gas and solid elemental carbon. In various embodiments as otherwise described herein, at least a portion of the H₂ of the first feed stream is turquoise hydrogen.

White hydrogen is naturally-occurring hydrogen which can be released through fracking. In various embodiments as otherwise described herein, at least a portion of the H₂ of the first feed stream is white hydrogen.

As described herein, the disclosed process, in various embodiments, includes two central reactions: the hydrogenation of CO₂ and methanol decomposition. As described herein, these reactions may be advantageously carried out in different spatial arrangements relative to each other. As known in the art, reaction zones are comprised of one or more catalyst beds, and one or more reaction zones may be within the same plant. Different plants are typically geographically separated so that feedstocks cannot be transferred between them without an intervening transportation and storage step. Accordingly, in various embodiments as otherwise described herein, the first reaction zone comprises a first reactor in which the hydrogenation catalyst is disposed, and the second reaction zone comprises a second reactor in which the methanol decomposition catalyst is disposed. In various embodiments as otherwise described herein, the first reaction zone comprises a first catalyst bed in which the hydrogenation catalyst is disposed, and the second reaction zone comprises a second catalyst bed in which the methanol decomposition catalyst is disposed. For example, in particular embodiments, the first catalyst bed, and the second catalyst bed are disposed within the same reactor, e.g., in catalyst stacks or catalyst bed sections. In particular embodiments, the first reactor is located in a first plant, and the second reactor is located in a second plant.

An example embodiment is shown in schematic view in FIG. 1, which depicts a process 100 that is contained within a single plant 141. First feed stream 111 comprising H₂ and CO₂ is conducted to hydrogenation reaction zone 110 (here, a hydrogenation reactor), and is contacted therein with a hydrogenation catalyst 113, under conditions appropriate to generate first product stream 112 comprising methanol. The first product stream will typically contain other substances as well. For example, in the embodiment of FIG. 1, the first product stream includes CO₂; at least a portion of the CO₂ is separated from the first product stream and recycled to the first feed stream 111 via CO₂ recycle 114. Similarly, the first product stream can include H₂ (e.g., unreacted H₂) and/or CO. Here, at least a portion of H₂ and/or CO is separated from the first product stream and recycled to the first feed stream 111 via H₂ and/or CO recycle stream 115. Any water present can be removed from the first product stream 112 by water separation stream 116, to give a predominantly dry second feed stream (121) and optionally the water feed stream 116 can be fed to an electrolysis reactor 160.

In the embodiment of FIG. 1, the remaining portions of the first product stream 112 (i.e., after the separations described above) are provided to the second feed stream 121. Second feed stream 121 includes at least a portion of the methanol of the first product stream, and is conducted to the methanol decomposition reaction zone 120 (here, a methanol decomposition reactor), in which it is contacted with methanol decomposition catalyst 123 to decompose at least a portion of the methanol to form a second product stream 122 comprising CO and H₂. The second feed stream 121 may also be augmented through the introduction of other gases, such as CO and/or H₂ through an augmenting feed (not shown). H₂ gas from the electrolysis reactor 160 is returned to the first feed stream by electrolysis H₂ stream 117.

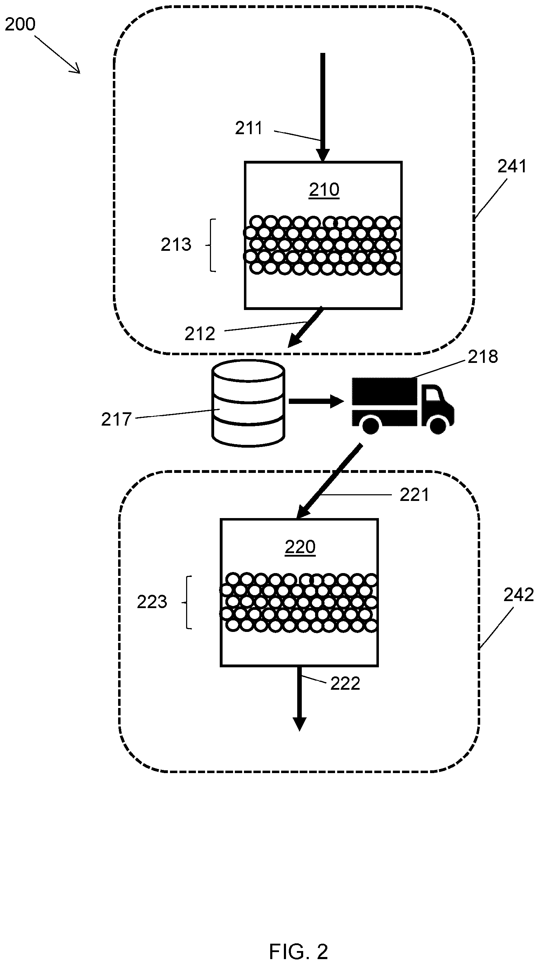

As noted above, methanol can be formed via hydrogenation of carbon dioxide in a first plant, then transported (e.g., via vehicle or pipeline) to a second plant where the methanol is decomposed and the resulting carbon monoxide and/or H₂ utilized in downstream processes.. An example of such an embodiment is shown in schematic view in FIG. 2. Here, the carbon dioxide hydrogenation step of process 200 is performed in a first plant 241. A first feed stream 211 comprising carbon dioxide and hydrogen is conducted to a hydrogenation reaction zone 210 (here, a hydrogenation reactor) containing CO₂ hydrogenation catalyst 213. The first feed stream 211 is contacted with the CO₂ hydrogenation catalyst 213 to hydrogenate at least a portion of the CO₂ to generate a first product stream 212 that includes methanol. First product steam 212 can be subjected to various separations as described herein, not shown, and then methanol of the first product stream can be stored for later use, and/or or transported to another plant. For example, in the embodiment of FIG. 1, at least a portion of the first product stream 212, the portion comprising methanol, is loaded into storage tank 217, then transported to second plant 243, here, by truck 218. The portion of the first product stream is provided to second feed stream 221, which is conducted to methanol decomposition reaction zone 220, in which it is contacted with methanol decomposition catalyst 223 to generate second product stream 222 comprising CO and H₂.

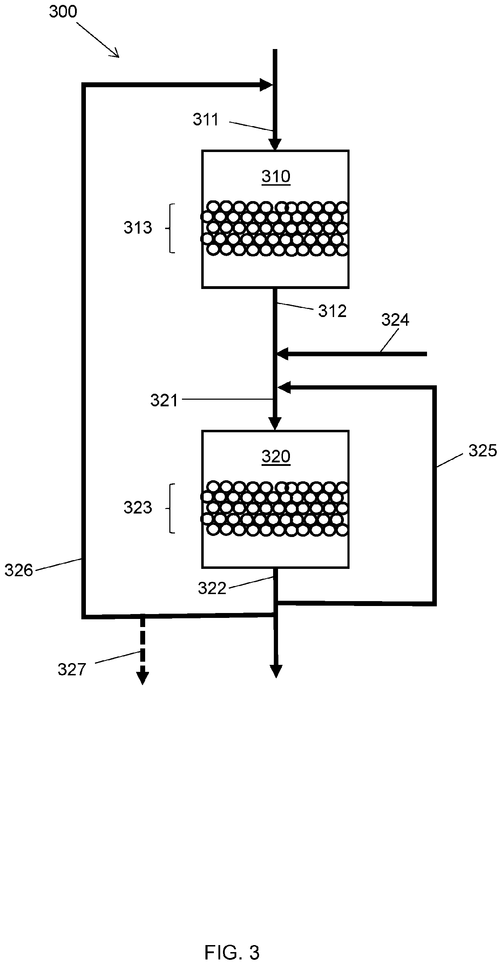

Another example embodiment is shown in schematic view in FIG. 3. Here, in process 300 first feed stream 311 is conducted to first reaction zone 310 and contacted with CO₂ hydrogenation catalyst 313 to hydrogenate at least a portion of CO₂ of the first feed stream to form first product stream 312 comprising methanol. First product stream 312 is conducted from the first reaction zone 310 and additional gas stream 324 is added, and the resulting mixture is provided as second feed stream 321. Second feed stream 321 conducted to methanol decomposition reaction zone 320 in which with methanol decomposition catalyst 323 to provide second product stream 322. Here, second product stream 322 is conducted from the methanol decomposition reaction zone, and unreacted methanol is substantially separated from the second product stream (e.g., at least 50%, at least 75%, at least 90% or at least 95%) and is recycled to the second feed stream 321 via methanol recycle 325. Notably, at least a portion of H₂ of the second product stream 322 can be separated and recycled to the first feed stream 311 via H₂ recycle 326. At least a portion of H₂ of the second product stream 322 may also or alternatively be provided to an external reactor or process through H₂ stream 327.

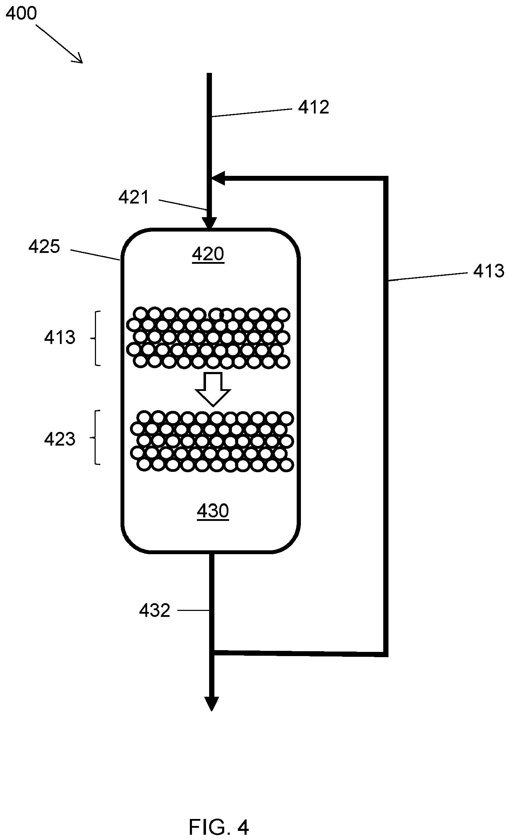

In some embodiments, the conversion of CO₂ to hydrocarbons through a methanol intermediate may be performed in a single reactor with various catalysts, for example, arranged in series. In the embodiment of FIG. 4, process 400 utilizes syngas source stream 412 which enters reactor 425 as first feed stream 421 at zone 420. The first feed stream 421 is contacted with hydrogenation catalyst 413, and then directly transmitted to methanol decomposition catalyst 423. The resulting gas mixture is withdrawn from zone 430 as second product stream 432. Recycle stream 413 withdraws CO₂ gas, in optional admixture with other gases, and recycles to CO₂ source stream 412.

Various exemplary embodiments of the disclosure include, but are not limited to the enumerated embodiments listed below, which can be combined in any number and in any combination that is not technically or logically inconsistent.
Embodiment 1. A process for producing syngas, the process comprising:
   providing a first feed stream comprising H₂ and CO₂;
   contacting the first feed stream with a hydrogenation catalyst (e.g., in a hydrogenation reaction zone) to hydrogenate at least a portion of the CO₂ to form a first product stream comprising methanol;
   providing a second feed stream comprising at least a portion of the methanol of the first product stream;
   contacting the second feed stream with a methanol decomposition catalyst (e.g., in a methanol decomposition reaction zone) to decompose at least a portion of the methanol to form a second product stream comprising syngas (e.g., comprising CO and H₂).
Embodiment 2. The process according to embodiment 1, wherein the first feed stream includes at least 10 mol% H₂, e.g., at least 20 mol% H₂, or at least 30 mol% H₂.
Embodiment 3. The process according to embodiment 1 or embodiment 2, wherein the first feed stream includes at least 5 mol% CO₂, e.g., at least 10 mol% CO₂, or at least 15 mol% CO₂.
Embodiment 4. The process according to any of embodiments 1-3, wherein a ratio of H₂:CO₂ of the first feed stream is at least 1:1, e.g.at least 1.5:1, at least 2:1, at least 2.5:1, at least 3:1, or at least 4:1.
Embodiment 5. The process according to any of embodiments 1-4, wherein the first feed stream further comprises one or more of CO, CH₄ and N₂.
Embodiment 6. The process according to any of embodiments 1-5, wherein the hydrogenation catalyst is a copper/zinc oxide catalyst, e.g., supported on aluminum oxide or zirconium oxide.
Embodiment 7. The process according to any of embodiments 1-6, wherein the contacting of the first feed stream with the hydrogenation catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.
Embodiment 8. The process according to any of embodiments 1-7, wherein the hydrogenation of CO₂ is performed with a selectivity of at least 50% for methanol, e.g., at least 65% or at least 80%.
Embodiment 9. The process according to any of embodiments 1-8, wherein the hydrogenation of CO₂ is performed with a selectivity of no more than 20% for methane, e.g., no more than 10%, or no more than 5%.
Embodiment 10. The process according to any of embodiments 1-9, wherein the hydrogenation of CO₂ is performed with a selectivity of no more than 20% for CO, e.g., no more than 10%, or no more than 5%.
Embodiment 11. The process according to any of embodiments 1-10, wherein the hydrogenation of CO₂ is performed with a conversion of CO₂ of at least 25%, e.g. at least 35%, e.g., at least 45% or at least 50%.
Embodiment 12. The process according to any of embodiments 1-11, wherein the first product stream includes at least 15 mol% methanol, e.g., at least 25 mol%, or at least 35 mol% methanol.
Embodiment 13. The process according to any of embodiments 1-12, wherein the first product stream includes no more than 10 mol% methane, e.g., no more than 5 mol% methane, or no more than 2 mol% methane.
Embodiment 14. The process according to any of embodiments 1-13, wherein the first product stream includes no more than 10 mol% CO, e.g., no more than 5 mol% CO, or no more than 2 mol% CO.
Embodiment 15. The process according to any of embodiments 1-14, further comprising separating at least a portion of water from the first product stream (e.g., at least 50%, at least 75%, or at least 90% of water in the first product stream).
Embodiment 16. The process of any of embodiments 1-15, wherein the portion of the first product stream that is included in the second feed stream has a water content of no more than 10 mol%, e.g., or no more than 2 mol%, or no more than 0.5 mol%.
Embodiment 17. The process according to any of embodiments 1-15, further comprising separating at least a portion of H₂ from the first product stream (e.g., at least 50%, or at least 60%, or at least 75%, or at least 90%, or at least 95% of H₂ in the first product stream).
Embodiment 18. The process of embodiment 17, further comprising recycling to the first feed stream at least a portion of the H₂ separated from the first product stream.
Embodiment 19. The process of any of embodiments 15-18, further comprising activating the methanol decomposition catalyst using at least a portion of the H₂ separated from the first product stream.
Embodiment 20. The process of any of embodiments 1-19, wherein the portion of the first product stream that is provided to the second feed stream includes no more than 50% of the hydrogen of the first product stream, e.g., no more than 25%, no more than 10%, or no more than 5% of the hydrogen of the first product stream.
Embodiment 21. The process of any of embodiments 1-20, wherein the contacting of the second feed stream with the methanol decomposition catalyst and the contacting of the first feed stream with the hydrogenation catalyst are performed in the same plant.
Embodiment 22. The process according to any of embodiments 1-21, wherein at least a portion of methanol of the first product stream is provided directly to the second feed stream.
Embodiment 23. The process according to embodiment 22, wherein the portion of the first product stream that is provided to the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 C, or 300-400 °C.
Embodiment 24. The process according to any of embodiments 1-20, wherein the contacting of the second feed stream with the methanol decomposition catalyst and the contacting of the first feed stream with the hydrogenation catalyst are performed in different plants.
Embodiment 25. The process according to any of embodiments 1-24, wherein at least a portion of methanol of the first product stream is stored (e.g., in one or more tanks) before provision to the second feed stream.
Embodiment 26. The process according to embodiment 25, wherein the stored portion of the methanol of the first product stream is stored for at least one day, e.g., at least two days or at least a week.
Embodiment 27. The process according to embodiment 25 or embodiment 26, wherein the stored portion of the methanol is transported at least 1 km during storage, e.g., at least 2 km or at least 10 km.
Embodiment 28. The process according to any of embodiments 1-27, wherein the second feed stream comprises at least 5 mol% methanol, e.g., at least 7.5% methanol, or at least 10 mol% methanol, or at least 15% methanol, or at least 20% methanol, or at least 25 mol% methanol.
Embodiment 29. The process according to any of embodiments 1-28, wherein the second feed stream further comprises one or more of H₂, CO, CH₄, CO₂ and N₂.
Embodiment 30. The process according to any of embodiments 1-29, wherein the second feed stream has a concentration of water of no more than 5 mol%, e.g., no more than 2 mol% or no more than 1 mol%.
Embodiment 31. The process according to any of embodiments 1-30, wherein the second feed stream has a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.
Embodiment 32. The process according to any of embodiments 1-31, wherein the methanol decomposition catalyst is a copper/zinc oxide catalyst, e.g., on alumina.
Embodiment 33. The process according to any of embodiments 1-32, wherein the contacting of the second feed stream with the methanol decomposition catalyst is performed at a temperature in the range of 200-500 °C, e.g., 200-450 °C, or 200-400 °C, or 200-350 °C, or 200-300 °C, or 250-500 °C, or 250-450 °C, or 250-400 °C, or 250-300 °C, or 300-500 °C, or 300-450 °C, or 300-400 °C.
Embodiment 34. The process according to any of embodiments 1-33, wherein the contacting of the second feed stream with the methanol decomposition catalyst is performed at a temperature within 75 °C of a temperature of the contacting of the first feed stream with the hydrogenation catalyst, e.g., within 50 °C.
Embodiment 35. The process according to any of embodiments 1-34, wherein the decomposition of methanol is performed with a carbon product selectivity of at least 50% for CO, e.g., at least 60%, or at least 70%, or at least 80%, or at least 90%.
Embodiment 36. The process according to any of embodiments 1-35, wherein the decomposition of methanol is performed with a selectivity of no more than 20% for CO₂, e.g., no more than 15%, no more than 10%, or no more than 5%.
Embodiment 37. The process according to any of embodiments 1-36, wherein the decomposition of methanol is performed with a conversion of methanol of at least 30%, e.g. at least 40%, or at least 50%, or at least 60%, or at least 65%, or at least 70%, or at least 75%.
Embodiment 38. The process according to any of embodiments 1-37, wherein the second product stream includes at least 20 mol% total of CO and H₂, e.g., at least 35 mol%, or at least 50 mol%, or at least 65 mol%.
Embodiment 39. The process according to any of embodiments 1-38, wherein the second product stream has a molar ratio of hydrogen to carbon monoxide in the range of 0.5:1 to 5:1, e.g., 1:1 to 3:1, 1.5:1 to 2.5:1.
Embodiment 40. The process according to any of embodiments 1-39, wherein the second product stream includes no more than 75 mol% methanol, e.g., no more than 60 mol% methanol, or no more than 50 mol% methanol, or no more than 25 mol% methanol.
Embodiment 41. The process according to any of embodiments 1-40, further comprising separating at least a portion of methanol from the second product stream (e.g., at least 50%, at least 75%, or at least 90% of methanol in the second product stream).
Embodiment 42. The process of embodiment 41, further comprising recycling to the second feed stream at least a portion of the methanol separated from the second product stream, e.g., at least 50%, or at least 75%, or at least 90%, or at least 95% of the methanol from the second product stream.
Embodiment 43. The process of any of embodiments 1-42, further comprising separating at least a portion of H₂ from the second product stream.
Embodiment 44. The process of embodiment 43, further comprising providing the H₂ separated from the second product stream to the first feed stream.
Embodiment 45. The process of any of embodiments 1-44, wherein at least part of the CO₂ of the first feed stream is from a renewable source.
Embodiment 46. The process of any of embodiments 1-45, wherein at least part of the CO₂ of the first feed stream is from direct air capture.
Embodiment 47. The process of any of embodiments 1-46 wherein at least part of the CO₂ of the first feed stream is captured from a manufacturing plant, e.g., a bioethanol plant, a steel plant or a cement plant.
Embodiment 48. The process of any of embodiments 1-47, wherein at least part of the H₂ of the first feed stream is from a renewable source.
Embodiment 49. The process of any of embodiment 1-48, wherein at least a portion of the hydrogen of the first feed stream is green hydrogen.
Embodiment 50. The process of any of embodiment 1-49, wherein at least a portion of the hydrogen of the first feed stream is blue hydrogen.
Embodiment 51. The process of any of embodiment 1-50, wherein at least a portion of the hydrogen of the first feed stream is grey hydrogen, black hydrogen, brown hydrogen, pink hydrogen, turquoise hydrogen, yellow hydrogen, and/or white hydrogen.
Embodiment 52. The process of any of embodiments 1-51, further comprising providing at least a portion of H₂ to the first feed stream by electrolysis of water.
Embodiment 53. The process of embodiment 52, wherein the electrolysis of water is performed using electricity at least partially derived from a renewable source.
Embodiment 54. The process of any of embodiments 1-53, wherein the first reaction zone comprises a first reactor in which the hydrogenation catalyst is disposed, and wherein the second reaction zone comprises a second reactor in which the methanol decomposition catalyst is disposed.
Embodiment 55. The process of any of embodiments 1-54, wherein the first reaction zone comprises a first catalyst bed in which the hydrogenation catalyst is disposed, and wherein the second reaction zone comprises a second catalyst bed in which the methanol decomposition catalyst is disposed.
Embodiment 56. The process of embodiment 55, wherein the first catalyst bed and the second catalyst bed are disposed within the same reactor in catalyst stacks or bed sections.

The particulars shown herein are by way of example and for purposes of illustrative discussion of various embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains various errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for producing syngas, the process comprising:
providing a first feed stream comprising H₂ and CO₂;
contacting the first feed stream with a hydrogenation catalyst (e.g., in a hydrogenation reaction zone) to hydrogenate at least a portion of the CO₂ to form a first product stream comprising methanol;
providing a second feed stream comprising at least a portion of the methanol of the first product stream;
contacting the second feed stream with a methanol decomposition catalyst (e.g., in a methanol decomposition reaction zone) to decompose at least a portion of the methanol to form a second product stream comprising syngas (e.g., comprising CO and H₂).

2. The process according to claim 1, wherein the first feed stream includes at least 10 mol% H₂, e.g., at least 20 mol% H₂, or at least 30 mol% H₂.

3. The process according to claim 1 or claim 2, wherein the first feed stream includes at least 5 mol% CO₂, e.g., at least 10 mol% CO₂, or at least 15 mol% CO₂.

4. The process according to any of claims 1-3, wherein a ratio of H₂:CO₂ of the first feed stream is at least 1:1, e.g.at least 1.5:1, at least 2:1, at least 2.5:1, at least 3:1, or at least 4:1.

5. The process according to any of claims 1-4, wherein the hydrogenation of CO₂ is performed with a selectivity of at least 50% for methanol, e.g., at least 65% or at least 80%.

6. The process according to any of claims 1-5, further comprising separating at least a portion of water from the first product stream (e.g., at least 50%, at least 75%, or at least 90% of water in the first product stream).

7. The process of any of claims 1-6, wherein the portion of the first product stream that is included in the second feed stream has a water content of no more than 10 mol%, e.g., or no more than 2 mol%, or no more than 0.5 mol%.

8. The process according to any of claims 1-7, wherein the second feed stream comprises at least 5 mol% methanol, e.g., at least 7.5% methanol, or at least 10 mol% methanol, or at least 15% methanol, or at least 20% methanol, or at least 25 mol% methanol.

9. The process according to any of claims 1-8, wherein the second feed stream has a concentration of water of no more than 5 mol%, e.g., no more than 2 mol% or no more than 1 mol%.

10. The process according to any of claims 1-9, wherein the contacting of the second feed stream with the methanol decomposition catalyst is performed at a temperature within 75 °C of a temperature of the contacting of the first feed stream with the hydrogenation catalyst, e.g., within 50 °C.

11. The process according to any of claims 1-10, wherein the decomposition of methanol is performed with a carbon product selectivity of at least 50% for CO, e.g., at least 60%, or at least 70%, or at least 80%, or at least 90%.

12. The process according to any of claims 1-11, wherein the decomposition of methanol is performed with a selectivity of no more than 20% for CO₂, e.g., no more than 15%, no more than 10%, or no more than 5%.

13. The process according to any of claims 1-12, wherein the second product stream includes at least 20 mol% total of CO and H₂, e.g., at least 35 mol%, or at least 50 mol%, or at least 65 mol%.

14. The process according to any of claims 1-13, wherein the second product stream has a molar ratio of hydrogen to carbon monoxide in the range of 0.5:1 to 5:1, e.g., 1:1 to 3:1, 1.5:1 to 2.5:1, or 1:5:1 to 3.5:1.

15. The process according to any of claims 1-14, further comprising separating at least a portion of H₂ from the second product stream, and providing the H₂ separated from the second product stream to the first feed stream.
